# EUROPÄISCHE PATENTSCHRIFT

(11) **EP 0 880 976 B1**
(45) Veröffentlichungstag und Bekanntmachung des Hinweises auf die Patenterteilung: **02.02.2005**
(21) Anmeldenummer: 98810401.4
(22) Anmeldetag: 05.05.1998
(51) Int. Cl.: A61M 25/04

(54) **Im Körper befestigbarer Katheter**
Catheter attachable in the body
Cathéter attachable dans le corps

(30) Priorität: 27.05.1997 CH 123997
(43) Veröffentlichungstag der Anmeldung: 02.12.1998
(73) Patentinhaber: Disetronic Licensing AG, 3401 Burgdorf (CH)
(72) Erfinder: Bestetti, G.E., Prof. Dr., 3098 Köniz (CH); Frei, Thomas, 3432 Lützelflüh (CH); Reinmann, Andreas, 3014 Bern (CH)
(74) Vertreter: Schwabe - Sandmair - Marx

(56) Entgegenhaltungen:
- WO-A-94/00177
- US-A- 4 057 066
- US-A- 5 000 732
- US-A- 5 061 255
- US-A- 5 242 415
- US-A- 5 628 780

## Beschreibung

Die Erfindung bezieht sich auf einen im menschlichen oder tierischen Körper befestigbaren Katheter gemäß dem Oberbegriff des Anspruchs 1.

Katheter, welche normalerweise längere Zeit im Körper belassen werden und durch welche Arzneimittel in menschliche oder tierische Blutgefäße geleitet werden, sind seit längerem bekannt. Solche Katheter können ohne Verbindungsglied durch die Haut in den Körper implantiert werden, so dass ein einziger Schlauch das Arzneimittel vom Arzneimittelbehälter bis zum Ausschüttungsort im Körper führt. Das Infektionsrisiko ist dabei aber besonders hoch. Häufig wird deshalb zwischen einem im Körper implantierten Katheter und einem außerhalb des Körpers gelegenen Infusionsschlauch ein sub- oder percutaner Portkörper angeordnet, welcher den implantierten Katheter und den Infusionsschlauch verbindet. Aus der Patentschrift US-A-5 306 255 ist ein subcutan implantierbarer Portkörper und aus der Patentschrift EP-B-0 302 076 ein percutan implantierbarer Portkörper bekannt.

Aus der Patentschrift US 5,061,255 ist ein Katheter bekannt, der an seiner Außenseite Vertiefungen aufweist, die ein Eindringen von Mikroorganismen und Ablagerungen in den Körper des Patienten verhindern. Dabei wird den Vertiefungen Energie zugeführt, die das Eindringen von Mikroorganismen verhindert.

Das US Patent 5,000,732 beschreibt einen Katheter, dessen eine Seite in dem Körper eines Lebewesens befestigt wird und dessen andere Seite mit einem Luer-Lock-Anschluss verbunden ist. Um ein Abgleiten des Katheters von dem Luer-Lock-Anschluss zu verhindern, sind beide mit einem chirurgischen Faden gegen Auseinandergleiten gesichert.

Bei bekannten implantierten Kathetern kann, sofern der Patient nicht daran gehindert wird sich zu bewegen, die Anordnung des Katheters in einem Blutgefäß dazu führen, dass der Katheter aus dem Gefäß gerissen wird oder aber der Katheter an der Blutgefäßinnenwand oder an der Eintrittsstelle des Katheters in das Blutgefäß reibt.

Hier will die Erfindung Abhilfe verschaffen. Der Erfindung liegt die Aufgabe zugrunde, einen befestigbaren Katheter zur längerfristigen Anordnung in einem schlauchförmigen Gebilde des menschlichen oder tierischen Körpers, welches ein Lumen aufweist, wie zum Beispiel ein Blutgefäß, ein Lymphgefäß oder die Umbilicalvene, zu entwickeln, welcher das Ausreiß- und Entzündungsrisiko vermindert, ohne den Patienten in der Bewegungsfreiheit einzuschränken.

Die Erfindung löst die gestellte Aufgabe mit einem Katheter, welcher die Merkmale des Anspruchs 1 aufweist.

Die durch die Erfindung erreichten Vorteile sind im Wesentlichen darin zu sehen, dass durch die Ausgestaltung des Katheters die Bewegungsfreiheit eines Patienten erhöht wird, unter gleichzeitiger Verminderung des Ausreiß- und Entzündungsrisikos.

Ein bevorzugtes Beispiel der Erfindung ist in den Figuren dargestellt.

Es zeigen:
- Fig. 1: Einen erfindungsgemäßen Katheter
- Fig. 2a: Eine Befestigungsart eines erfindungsgemäßen Katheters
- Fig. 2b: Eine bevorzugte Befestigungsart eines erfindungsgemäßen Katheters
- Fig. 3: Eine Koppelungsart eines erfindungsgemäßen Katheters mit einem Gehäuse.

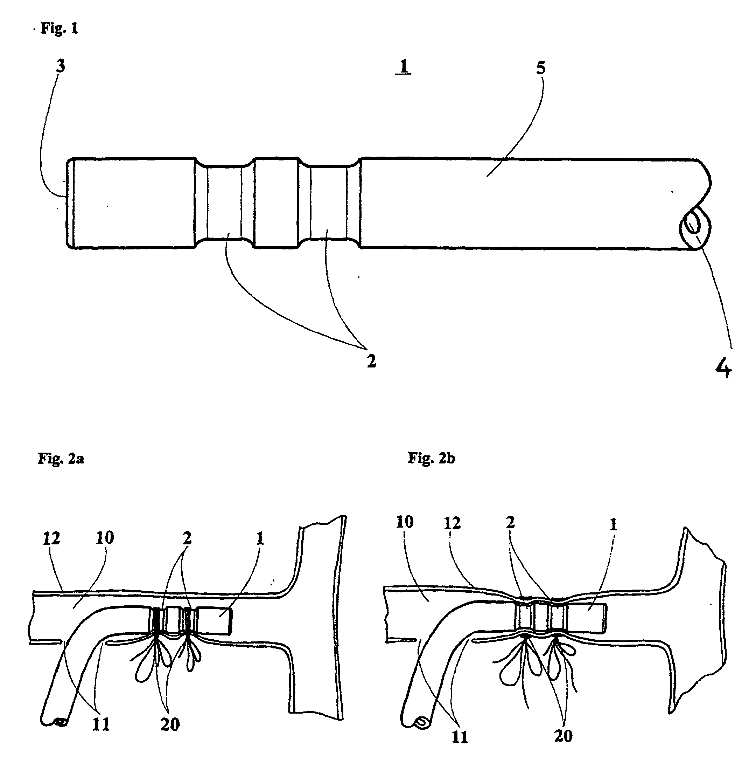

Wie in der Figur 1 dargestellt, besteht der erfindungsgemäße Katheter 1 aus einem zylindrischen Schaft 5, einem im Inneren des Katheters angeordneten durchgehenden Arzneimittelkanal 4 und einem Auslass 3. Gegen den Auslass 3 sind zwei radiale Rillen 2 angeordnet. Aus herstellungstechnischen Gründen wird der Auslass 3 mit den Rillen 2 als einstückiges, zusätzliches Element auf einen bestehenden Katheter 1 aufgeschweißt. Vorzugsweise wird deshalb der Außendurchmesser des aufgeschweißte Auslassstückes größer sein, als der übrige schlauchförmige Teil des Katheters 1.

Aus den Figuren 2a und 2b ist ersichtlich, dass der Katheter 1 in einem schlauchförmigen Gebilde 10, welches ein Lumen aufweist, befestigt wird. Der Katheter 1 wird dabei durch eine künstlich geschaffene Öffnung 11 in der Schlauchwand 12 geschoben. Das schlauchförmige Gebilde 10 ist zum Beispiel ein noch funktionierendes Blut- oder Lymphgefäß, oder auch ein ausgedorrtes Gefäß, wie zum Beispiel die Umbilicalvene. Der Katheter 1 wird in das schlauchförmige Gebilde 10 geschoben und anschließend mit Hilfe einer biokompatiblen Schnur 20 befestigt. Wie in Figur 2b dargestellt, wird die Schnur 20 über einer radialen Rille 2 des Katheters 1, derart um das schlauchförmige Gebilde 10 gewickelt, dass die Schnur 20 die Schlauchwand 12 in die radiale Rille 2 presst. Die Schnur 20 kann aber auch, wie in Figur 2a gezeigt, die Schlauchwand 12 nur teilweise umwickeln und die Schlauchwand 12 an einer bestimmten Stelle durchbohren, so dass die Schnur 20 innerhalb des schlauchförmigen Gebildes 10 in der radialen Rille 2 verläuft und außerhalb des schlauchförmigen Gebildes 10 die Schlauchwand 12 in die radiale Rille 2 presst. Dies hat den Vorteil, dass das Lumen nicht abgeschlossen wird.

In den dargestellten Ausführungsformen weist der Katheter 1 aus Sicherheitsgründen zwei radiale Rillen 2 auf.

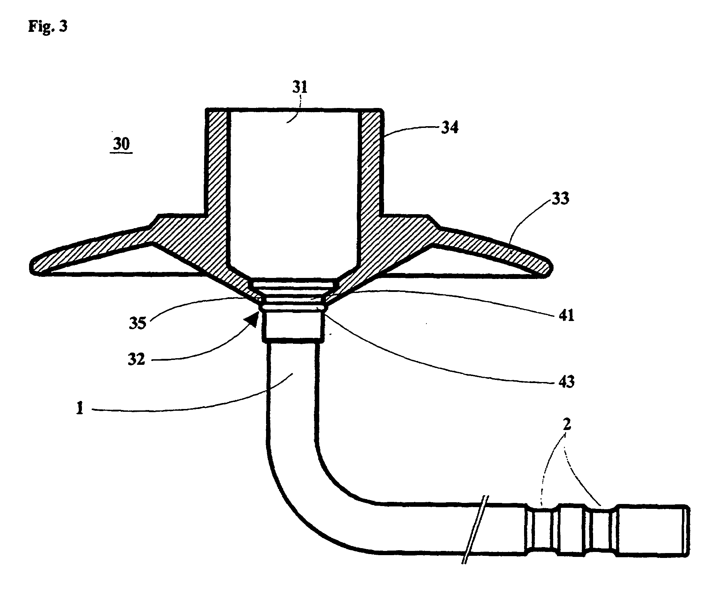

Wie in Fig. 3 dargestellt, kann der Katheter 1 mit einem, gegen das Körperinnere kegelförmig auslaufenden, Portkörper 30 verbunden werden. Solche Portkörper dienen der Verbindung eines außerhalb des menschlichen oder tierischen Körpers gelegenen Infusionsschlauches, mit einem im Inneren des menschlichen oder tierischen Körpers gelegenen Katheter 1. Der Portkörper 30 besteht aus einer hohlzylindrischen Hülse 34 und einem daran angeordneten radialen Verankerungsteller 33. Der Portkörper 30 weist eine erste Öffnung 31 gegen die Außenwelt und eine zweite Öffnung 32 gegen das Körperinnere auf. Die zweite Öffnung 32 befindet sich am kegelförmig auslaufenden Ende des Portkörpers 30. Durch die zweite Öffnung 32 des Portkörpers wird eine Hülsenkante 35 gebildet.

In der bevorzugten Ausführungsart weist der Katheter 1 am dem Auslass 3 gegenüberliegenden Ende einen trichterförmigen Katheterkopf 41 auf, wobei dessen Grundlinie einen größeren Durchmesser aufweist, als die zweite Öffnung 32 des Portkörpers 30. Unterhalb des Katheterkopfes 41 ist eine radiale Rippe 43 angeordnet, deren Durchmesser ebenfalls größer ist als die zweite Öffnung 32 des Portkörpers 30.

Der Katheter 1 wird derart mit dem Portkörper 30 verbunden, dass die Hülsenkante 35 des Portkörpers 30 zwischen der radialen Rippe 43 des Katheters 1 und dem Katheterkopf 41 zu liegen kommt. Dabei bewirkt die Trichterform des Katheterkopfes 41 dass sich der Katheterkopf 41 an der Kante 35 verkeilt und damit eine Bewegung des Katheters 1 gegen das Körperinnere verhindert, während die radiale Rippe 43 eine Bewegung des Katheters 1 in die andere Richtung verhindert.

Der Katheter 1 kann dabei entweder als Arzneimittelkatheter oder lediglich als Führungskatheter ausgebildet sein. Die beiden Katheterarten unterscheiden sich dadurch, dass der Führungskatheter in seinem Inneren nicht einen durchgehenden Arzneimittelkanal 4, sondern einen Kanal 4 zur Durchleitung eines Arzneimittelkatheters aufweist.

## Patentansprüche

1. Schlauchförmiger Katheter (1) zur Befestigung in einem schlauchförmigen Gebilde (10) des menschlichen oder tierischen Körpers, welches ein Lumen aufweist, wobei der Katheter (1) über wenigstens eine radiale Rille (2) verfügt, die gegen den Auslass des Katheters angeordnet ist, **dadurch gekennzeichnet, dass** der Katheter (1) mit einem Portkörper (30) verbunden ist und somit mit dem Portkörper (30), der einer Verbindung des Katheters (1) mit einem Infusionsschlauch dient, verwendet wird.

2. Katheter nach Anspruch 1, **dadurch gekennzeichnet, dass** mehrere Rillen (2) gegen den Auslass (3) des Katheters angeordnet sind.

3. Katheter nach Anspruch 1 oder 2, **dadurch gekennzeichnet, dass** der Katheter (1) über ein dem Auslass (3) gegenüberliegenden trichterförmigen Kopfteil (41) verfügt.

4. Katheter nach Anspruch 3, **dadurch gekennzeichnet, dass** unterhalb des Kopfteils (41) eine radiale Rippe (43) angeordnet ist.

5. Katheter nach einem der vorhergehenden Ansprüche, **dadurch gekennzeichnet, dass** die Verbindung mit einem Portkörper (30) durch Verkeilung des trichterförmigen Kopfteils (41) mit einer Hülsenkante (35), welche durch eine Öffnung (32) des Portkörpers (30) gebildet wird, erfolgt.

6. Katheter nach den Ansprüchen 4 und 5, **dadurch gekennzeichnet, dass** die Hülsenkante (35) zwischen der radialen Rippe (43) und dem trichterförmigen Kopfteil des Katheters (1) liegt.

7. Katheter nach einem der Ansprüche 1 bis 6, **dadurch gekennzeichnet, dass** die radialen Rillen (2) ein Einpressen der Schlauchwand (12) des schlauchförmigen Gebildes (10) mittels biokompatibler Befestigungsmittel (20) ermöglichen.

8. Katheter nach Anspruch 7, **dadurch gekennzeichnet, dass** das biokompatible Befestigungsmittel (20) ein Faden ist.

9. Katheter nach einem der vorhergehenden Ansprüche, **dadurch gekennzeichnet, dass** ein Auslassstück mit radialen Rillen (2) auf den schlauchförmigen Katheter (1) aufgeschweißt wird.

10. Katheter nach einem der Ansprüche 1 bis 9, **dadurch gekennzeichnet, dass** die Befestigung in einem Blut- oder Lymphgefäß oder einer Umbilicalvene erfolgt.

## Claims

1. A tubular catheter (1) to be attached in a tubular structure (10) of the human or animal body, said structure comprising a lumen and said catheter (1) comprising at least one radial groove (2) arranged towards the outlet of the catheter, **characterised in that** the catheter (1) is connected to a port body (30) and is thus used with the port body (30), which serves to connect the catheter (1) to an infusion tube.

2. The catheter as set forth in claim 1, **characterised in that** a number of grooves (2) are arranged towards the outlet (3) of the catheter.

3. The catheter as set forth in claim 1 or 2, **characterised in that** the catheter (1) comprises a funnel-shaped head portion (41) opposite the outlet (3).

4. The catheter as set forth in claim 3, **characterised in that** a radial groove (43) is arranged below the head portion (41).

5. The catheter as set forth in any one of the preceding claims, **characterised in that** the connection to a port body (30) is formed by wedging the funnel-shaped head portion (41) to a sleeve edge (35) formed by an opening (32) of the port body (30).

6. The catheter as set forth in claims 4 and 5, **characterised in that** the sleeve edge (35) lies between the radial groove (43) and the funnel-shaped head portion of the catheter (1).

7. The catheter as set forth in any one of claims 1 to 6, **characterised in that** the radial grooves (2) enable the tube wall (12) of the tubular structure (10) to be pressed in by means of biocompatible attaching means (20).

8. The catheter as set forth in claim 7, **characterised in that** the biocompatible attaching means (20) is a thread.

9. The catheter as set forth in any one of the preceding claims, **characterised in that** an outlet piece comprising radial grooves (2) is fused onto the tubular catheter (1).

10. The catheter as set forth in any one of claims 1 to 9, **characterised in that** the attachment is made in a blood or lymph vessel or an umbilical vein.

## Revendications

1. Cathéter (1) de forme tubulaire à fixer dans une formation tubulaire (10) du corps humain ou animal qui comprend une lumière, le cathéter (1) disposant d'au moins une rainure radiale (2) qui est disposée à proximité de la sortie du cathéter, **caractérisé par le fait que** le cathéter (1) est relié à un corps de chambre implantable (30) et est ainsi utilisé avec le corps de chambre implantable (30) qui sert à relier le cathéter (1) au tuyau de perfusion.

2. Cathéter selon la revendication 1, **caractérisé en ce que** plusieurs rainures (2) sont disposées à proximité de la sortie (3) du cathéter.

3. Cathéter selon la revendication 1 ou 2, **caractérisé en ce qu'**il est pourvu d'un élément d'extrémité (41) de forme conique situé à l'opposé de la sortie (3).

4. Cathéter selon la revendication 3, **caractérisé en ce qu'**une rainure (43) radiale est disposée sous l'élément d'extrémité (41).

5. Cathéter selon une des revendications précédentes, **caractérisé en ce que** la liaison au corps de chambre implantable (30) se produit via le blocage de l'élément d'extrémité (41) de forme conique avec un bord de manchon (35) lequel est formé par une ouverture (32) du corps de chambre implantable (30).

6. Cathéter selon la revendication 5, **caractérisé en ce que** le bord de manchon (35) se situe entre la rainure radiale (43) et l'élément d'extrémité de forme conique du cathéter (1).

7. Cathéter selon une des revendications 1 à 6, **caractérisé en ce que** les rainures radiales (2) permettent un emmanchement de la paroi du tuyau (12) de la formation tubulaire (10) à l'aide d'un moyen de fixation (20) biocompatible.

8. Cathéter selon la revendication 7, **caractérisé en ce que** le moyen de fixation (20) biocompatible est un fil.

9. Cathéter selon une des revendications précédentes, **caractérisé en ce qu'**un élément de sortie est superposé par soudure avec les rainures radiales (2) sur le cathéter (1) de forme tubulaire.

10. Cathéter selon une des revendications 1 à 9, **caractérisé en ce que** la fixation se produit dans un vaisseau lymphatique ou sanguin ou une veine ombilicale.
